(19)

Europäisches Patentamt
European Patent Office
Office européen des brevets

(11) **EP 4 732 828 A1**

(12) **EUROPEAN PATENT APPLICATION**
published in accordance with Art. 153(4) EPC

(43) Date of publication:
**29.04.2026 Bulletin 2026/18**

(21) Application number: 24809880.8

(22) Date of filing: **24.05.2024**

(51) International Patent Classification (IPC):
**A61K 8/92** (2006.01)     **A61K 8/34** (2006.01)
**A61K 8/84** (2006.01)     **A61K 8/41** (2006.01)
**A61K 8/60** (2006.01)     **A61Q 5/12** (2006.01)

(52) Cooperative Patent Classification (CPC):
**A61K 8/34; A61K 8/84; A61K 8/92;** A61K 8/41;
A61K 8/60; A61Q 5/12

(86) International application number:
**PCT/BR2024/050212**

(87) International publication number:
**WO 2024/239089 (28.11.2024 Gazette 2024/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.05.2023 BR 102023010164**

(71) Applicant: **Ashland Comércio de Especialidades
Químicas do
Brasil Ltda.
05321-900 São Paulo (BR)**

(72) Inventors:
• **BAZITO, Alexandra**
  **03185-020 São Paulo (BR)**
• **KUMAYAMA, Tatiana Miyashiro**
  **09751-250 São Paulo (BR)**
• **BRENNER, Liliana Carole**
  **05716-090 São Paulo (BR)**

(74) Representative: **KATZAROV S.A.**
  **Geneva Business Center
  12 Avenue des Morgines
  1213 Petit-Lancy (CH)**

(54) **ACTIVE COSMETIC SYSTEM, USE OF THE ACTIVE COSMETIC SYSTEM, COSMETIC COMPOSITION AND METHOD OF COSMETIC TREATMENT OF THE HAIR FIBRES AND/OR SCALP**

(57)     The present invention relates to an active cosmetic system, as well as to a cosmetic composition comprising said system. In addition, the present invention relates to the use of an active cosmetic system as defined herein for the preparation of a cosmetic composition and to a method of cosmetic treatment of hair fibres or the scalp. The present invention is in the field of cosmetics, hair and scalp treatment.

[Fig. 1]

1% Wet Combability with rinse Finos in bleached hair - Total Work Joules

## Description

### Field of the Invention

The present invention relates to an active cosmetic system, as well as a cosmetic composition comprising said system. Additionally, the present invention relates to the use of an active cosmetic system as defined herein for the preparation of a cosmetic composition, and a method for the cosmetic treatment of hair fibers. The present invention is situated in the cosmetic area, hair, and scalp treatment.

### Background of the Invention

Pure vegetable oils, such as coconut oil, babassu oil, baobab oil, sesame, buriti, among others; and/or silicones and/or modified esters, are ingredients usually employed in cosmetic applications with the objective of obtaining soft and shiny hair. To improve hair combability, poorly biodegradable quaternary compounds are usually used.

Although vegetable oils are known to improve hair frizz and maintain curls, when used in their pure form, they present a heavy and slightly unpleasant sensory feel to the touch, and also do not maintain a very natural appearance of the hair and curls. Furthermore, they are difficult to remove from the hair fiber, and also when used in cleaning systems, such as shampoos, they destabilize the system if used at the appropriate percentage for the benefit of shine and curl maintenance. Additionally, vegetable oils can also present an unpleasant odor and are subject to oxidation.

Oh the other hand, hydrophobic silicones applied to obtain shine, anti-frizz, and combability of the locks, present a better sensory feel on the hair, but leave the hair heavy and lifeless, they are difficult to remove from the hair fiber, and they are also difficult to incorporate into systems such as shampoos.

C16 chain quaternary compounds are known to promote conditioning, however, they are irritating to the scalp and promote frizz, and with this type of material, wet conditioning is obtained. In the case of longer chains, such as C22, dry hair conditioning is possible, they improve frizz, but these materials have a high melting point which impairs the shine of the hair fiber. In addition, they also deposit on the hair fiber over time, which makes it lifeless over time.

The conditioning and curl styling products currently known on the market have deficiencies:

- They promote deposition on the hair fiber, which over time changes its properties, causing frizz and lack of shine. Furthermore, conditioning products are generally petroleum-derived or contain long-chain materials in their composition, which, due to deposition on the fiber, impair shine, as they become solid at room temperature;
- They negatively interfere with the scalp microbiota. The existing nitrogen chains make the product's biodegradability difficult and also interfere with the type and quantity of commensal bacteria. Commensal bacteria are known to play an important role in maintaining the health of the organism, helping to prevent the colonization of pathogenic bacteria that can cause diseases. They also contribute to digestion, nutrient absorption, and vitamin synthesis;
- As they are products that do not comprise sequential chains, they hinder the styling of the hair fiber, not providing durability in the shape of the curl or the fiber itself;
- They have an unpleasant odor and are subject to oxidation, impairing the benefit-to-aesthetic ratio; and
- They are generally toxic to aquatic life and the environment.

The present invention aims to provide a cosmetic system that is not toxic to the environment and that is capable of promoting all desired cosmetic functionalities to the hair and scalp simultaneously and in a single product, namely, conditioning, shine, softness, curl control, and strand alignment, without the occurrence of deposition on the hair fibers and which presents ease of removal and possibility of use in cleaning systems, such as shampoo, without interfering with the transparency of the system or its stability.

### Summary of the Invention

The present invention relates to an active cosmetic system, particularly in the form of a microemulsion, which comprises a unique combination of cosmetic ingredients.

An object of the present invention is the use of the active cosmetic system for the preparation of cosmetic compositions for hair and/or scalp care.

An additional object of the present invention is to provide a cosmetic composition comprising the cosmetic system described herein.

Another object of the invention is a method for the cosmetic treatment of hair fibers and/or the scalp.

These and other objects of the invention will be immediately appreciated by those skilled in the art, and will be described in sufficient detail for their reproduction in the following description.

## Brief Description of the Figures

Figures 1, 5, and 13 illustrate graphs that present comparative results regarding a wet combability test of a control sample versus samples treated with the active systems of the invention.

Figures 2, 6, and 14 illustrate graphs that present comparative results regarding a dry combability test of a control sample versus samples treated with the active systems of the invention.

Figures 3 and 11 illustrate graphs that present comparative results regarding a luminosity test of a control sample versus samples treated with the active systems of the invention.

Figures 4 and 12 illustrate graphs that present comparative results regarding a shine test of a control sample versus samples treated with the active systems of the invention.

Figures 7a-d and Figures 9 a-c illustrate the results of the effect on volume and frizz in curly hair fiber samples treated with the active systems of the invention versus a control sample.

Figures 8a-f and Figures 10a-d illustrate the results of the effect on volume, frizz, and styling of curl lock samples treated with the active systems of the invention versus a control sample.

Figure 15 represents the particle size and distribution measurement of the active system of the invention comprising vegetable oils.

Figure 16 illustrates a microscopic analysis of control systems, the invention (comprising vegetable oils), and pure vegetable oils.

## Detailed Description of the Invention

The active cosmetic system of the present invention comprises: at least one emulsifying agent, at least one conditioning agent, at least one humectant agent, and any other cosmetically acceptable ingredients.

By active cosmetic system, according to the present invention, is meant an active system with a high degree of vegetalization and biodegradability for hair fibers, for deposition and homogeneous delivery of vegetable oils to hair fibers, with the function of improving the softness, shine, hydration, nutrition, frizz, curl styling, and appearance of kinky, fine, and damaged hair and all hair that needs better shine.

The cosmetic system particularly comprises a primary emulsifying agent and a secondary emulsifying agent.

The primary emulsifying agent is an ingredient that should be useful in the personal care and cosmetic industry, which acts as an emulsifier and/or stabilizer and/or thickener, to improve the texture and stability of the formulation. The emulsifying agents are selected from the group comprising: sucrose stearate, lauryl sucrose, cetyl sucrose, 100% plant-derived compounds such as sorbitan olivate, polyglyceryl-6 olivate, polyglyceryl compounds in general, lecithin, or a mixture thereof. Sucrose stearate is particularly preferred.

The secondary emulsifying agent is a plant-derived cosmetic ingredient, which has the function of improving the absorption of other ingredients in the formulation, enhancing the effects of other active ingredients in the cosmetic product, and also helping to strengthen the hair and improve its texture and shine, and may also act as a moisturizing and conditioning agent. The secondary emulsifying agent particularly contributes to increasing lubricity and maintaining the transparency of the system. The secondary emulsifying agents useful in the present invention are selected from the group comprising hydrolyzed wheat protein, hydrolyzed rice protein, soy extract or derivative, hydrolyzed soy protein, glyceryl/octyldodecanol soy oil ester, or a mixture thereof. The ingredient Antaron soy, marketed by the company Ashland, which is a glyceryl/octyldodecanol soy oil maleate ester, is preferred.

The conditioning agent is water-soluble and preferably partially synthetic. It is an ingredient useful in the personal care and cosmetic industry, possessing moisturizing and lubricating properties, and that helps to improve hair texture and softness. Ingredients derived from fatty acid esters, such as vegetable oils, vegetable butters, proteins, and quaternary compounds such as quaternium 22, such as Ceraphyl 60, marketed by the company Ashland, may be used.

The humectant agent is an ingredient useful for cosmetic hair products, whose main function is to attract water to the hair, keeping it hydrated and soft, it can also help to control frizz, reducing the unruly appearance and giving a more disciplined and soft appearance. It is important to note that excessive use of this ingredient may have the opposite effect, making the hair heavy and oily. The humectant agents useful in the present invention are selected from the group comprising propanediol, sorbitol, glycerin, or a mixture thereof. Glycerin is particularly preferred.

The active cosmetic system comprises:

- about 0.5-11%, preferably 3-6% based on the total weight of the system, of at least one emulsifying agent;
- about 1-5%, preferably 2-4% based on the total weight of the system, of at least one conditioning agent;
- about 80-97%, preferably 90-95% based on the total weight of the system, of at least one humectant agent.

In a preferred embodiment, the active cosmetic system comprises:

- 2-4%, based on the total weight of the system, of a primary emulsifying agent;
- 1-2%, based on the total weight of the system, of a secondary emulsifying agent;
- 2-4%, based on the total weight of the system, of a conditioning agent; and
- 90-95%, based on the total weight of the system, of a humectant agent.

The cosmetic system may additionally comprise about 0.5-3.0% by weight based on the total weight of the system, of saturated and unsaturated vegetable oils.

Saturated vegetable oils are oils that predominantly contain saturated fatty acids in their composition. Saturated fatty acids are fat molecules that do not have double bonds between their carbon atoms, which gives them a rigid molecular structure. Saturated vegetable oils are found in foods such as coconut oil, palm oil, cocoa butter, and babassu oil, among others.

Unsaturated vegetable oils are oils that predominantly contain unsaturated fatty acids in their composition. Unsaturated fatty acids are fat molecules that have at least one double bond between their carbon atoms, which gives them a more flexible molecular structure. Unsaturated vegetable oils are found in foods such as olive oil, canola oil, sunflower oil, soy oil, peanut oil, sesame, avocado, nuts, buriti oil, and baobab oil, among others.

The ratio of unsaturated oils to saturated oils in the composition of the cosmetic system of the present invention varies from 10:90, particularly from 40:60, preferably 25:75.

Advantageously and surprisingly, the microemulsion formed with the presence of vegetable oils does not present odor nor is it susceptible to oxidation as occurs with pure vegetable oils.

Coconut and babassu oils, or a combination thereof (as examples of saturated vegetable oils) and baobab, buriti, and sesame oils, or a combination of two or more thereof (as examples of unsaturated vegetable oils), are particularly preferred.

The cosmetic system of the present invention is in the form of a microemulsion, whose particle distribution in the medium is less than 1μm, particularly between 0.5 μm - 1 μm.

The active cosmetic system disclosed herein has a surprisingly stable structure and a homogeneous particle distribution.

The cosmetic system comprising vegetable oil/glycol microemulsion with vegetable oils acts as an active system useful for hair and scalp treatment, with the function of improving the softness and appearance of kinky, fine, and damaged hair fibers and all hair that needs better shine.

The active cosmetic system can be added as an active ingredient to various cosmetic compositions and formulations for hair and/or scalp care, such as creams, lotions, soaps, shampoos, conditioners, hair masks, leave-on products, split end repairers, and other personal care products for the hair and/or scalp.

The active system of the invention enables the deposition and homogeneous delivery of vegetable oils to hair fibers with the function of improving softness, shine, hydration, nutrition, frizz, curl styling, and the appearance of the fiber. In other words, it is an active ingredient with a high degree of vegetalization and biodegradability for hair fibers with the function of improving the softness and appearance of kinky, fine, and damaged hair fibers and all hair that needs better shine.

Therefore, an object of the present invention is a method for the cosmetic treatment of hair fibers and/or the scalp, which comprises the application of the active system, or a cosmetic composition comprising the system, onto the hair fibers and/or the scalp. The method of cosmetic treatment may comprise steps such as washing, drying, maintaining the product on the hair fibers and/or the scalp, rinsing, among other steps routine for a person skilled in the art.

The following examples illustrate preferred embodiments according to the present invention, without imposing any limitations on its scope.

## Examples

### Example 1 - Active cosmetic system

Active cosmetic systems were formulated according to the invention. The formulations are described in the tables below:

[Table 1] - System without vegetable oils

| Components | Formulation 1 % by weight of the final formulation | Formulation 2 % by weight of the final formulation |
|---|---|---|
| Sucrose stearate | 4.00 | 2.00 |

(continued)

| Components | Formulation 1 % by weight of the final formulation | Formulation 2 % by weight of the final formulation |
|---|---|---|
| Antaron soy | 2.00 | 1.00 |
| Ceraphyl 60 | 4.00 | 2.00 |
| Glycerin | 90.00 | 95.00 |
| Total | 100.00 | 100.00 |

[Table 2] - System with vegetable oils

| Components | Formulation 3 % by weight of the final formulation |
|---|---|
| Sucrose stearate | 2.00 |
| Antaron soy | 1.00 |
| Ceraphyl 60 | 2.00 |
| Coconut oil or babassu oil | 1.5 |
| Baobab oil or buriti oil or sesame | 0.5 |
| Glycerin | 93.00 |
| Total | 100.00 |

## Example 2 - Cosmetic formulations

Cosmetic compositions of shampoo and rinse-off conditioner were formulated with the addition of the active cosmetic system of the invention.

[Table 3] - Shampoo

| Ingredients (Commercial Name / INCI) | | 12925-24-19 Control (%) | 12925-24-33 Invention (%) | 112925-24-31 Invention (%) |
|---|---|---|---|---|
| **Phase A** | | | | |
| Water | Water | 60.1 | 59.60 | 59.10 |
| Disodium EDTA | Disodium EDTA | 0.10 | 0.10 | 0.10 |
| 27% Sodium lauryl sulfate | Sodium lauryl sulfate | 30.00 | 30.00 | 30.00 |
| **Phase B** | | | | |
| 30% Cocamidopropyl betaine | Cocamidopropyl betaine | 6.00 | 6.00 | 6.00 |
| Ash1410022-1 System of the invention Formulation 3 (babassu oil + buriti oil) | | - | 0.50 | 1.00 |
| **Phase C** | | | | |
| Optiphen | Phenoxyethanol / caprylyl glycol | 0.80 | 0.80 | 0.80 |
| Sodium chloride | Sodium chloride | 3.00 | 3.00 | 3.00 |

Results: The shampoo formulation containing Ash1410022-1 (Formulation system 3 of the invention, as defined in example 1) showed good sensory performance. In addition to transparency, the panelists felt good lubricity and smoothness during and after rinsing with shampoo.

The shampoo formulation containing Ash1410022-1 maintained the transparent appearance of the shampoo at both system concentrations (0.5% and 1%). The shampoo formulation containing Ash1410022-1 did not have a negative impact on viscosity and no unwanted incorporation of bubbles occurred during the shampoo preparation procedure. According to technical and non-technical panelists, the shampoo formula with 1% Ash1410022 performed better in all attributes, namely, foam volume, foam creaminess, wet detangling, softness, and transparency.

[Table 4] - Rinse-off Conditioner

| Ingredients (Commercial Name | INCI) | | 12925-27 Control (%) | 12925-27-10 Invention (%) | 12925-27-12 Invention (%) |
|---|---|---|---|---|
| **Phase A** | | | | |
| Water | Water | 82.61 | 81.61 | 82.11 |
| Natrosol™ 330 Plus | Cetyl hydroxyethylcellulose | 0.15 | 0.15 | 0.15 |
| Disodium EDTA | Disodium EDTA | 0.10 | 0.10 | 0.10 |
| Glycerin | Glycerin | 2.00 | 2.00 | 2.00 |
| 85% Lactic acid | 85% Lactic acid | 0.20 | 0.20 | 0.20 |
| Stearamidopropyl dimethylamine | Stearamidopropyl dimethylamine | 0.70 | 0.70 | 0.70 |
| **Phase B** | | | | |
| Behentrimonium chloride | Behentrimonium chloride | 1.45 | 1.45 | 1.45 |
| Cetearyl alcohol | Cetearyl alcohol | 5.50 | 5.50 | 5.50 |
| **Phase C** | | | | |
| Ceraphyl™ 60 | Quaternium-22 | 3.33 | 3.33 | 3.33 |
| Ceraphyl™ 55 | Tridecyl neopentanoate | 2.50 | 2.50 | 2.50 |
| Fiberhance™ BM solution | Hydroxypropylglucona mide (and) hydroxypropylammoniu m gluconate | 1.00 | 1.00 | 1.00 |
| Optiphen™ BSBW | Benzyl alcohol (and) sodium benzoate (and) potassium sorbate | 0.30 | 0.30 | 0.30 |
| **Phase D** | | | | |
| 85% Lactic acid | 85% Lactic acid | 0.16 | 0.16 | 0.16 |
| Ash1410022-1 | Formulation 3 (babassu oil + buriti oil) | - | 1.00 | 1.00 |

Results: The conditioner formulation containing Ash1410022-1 (Formulation system 3 of the invention, as defined in example 1) did not have a negative impact on viscosity. The technical panel evaluated (in use) the rinse-off conditioner with 1% Ash1410022-1, which showed better performance in all attributes (wet softness, slipperiness, wet detangling, dry softness, dry detangling, volume, and frizz). The addition of the functional system Ash1410022-1 to rinse-off conditioners increased the properties listed below when compared to the control: anti-frizz, volume control, softness, wet and dry detangling, slipperiness/lubricity.

**Example 3** - **Hair fiber combability force properties**

The effect of a treatment with the active cosmetic system of the invention comprising a combination of oils, also called Activated Oil Finos version here (babassu oil/buriti oil ratio of 75/25), was tested on the combability force quantified by the total work through Dia-Stron analysis.

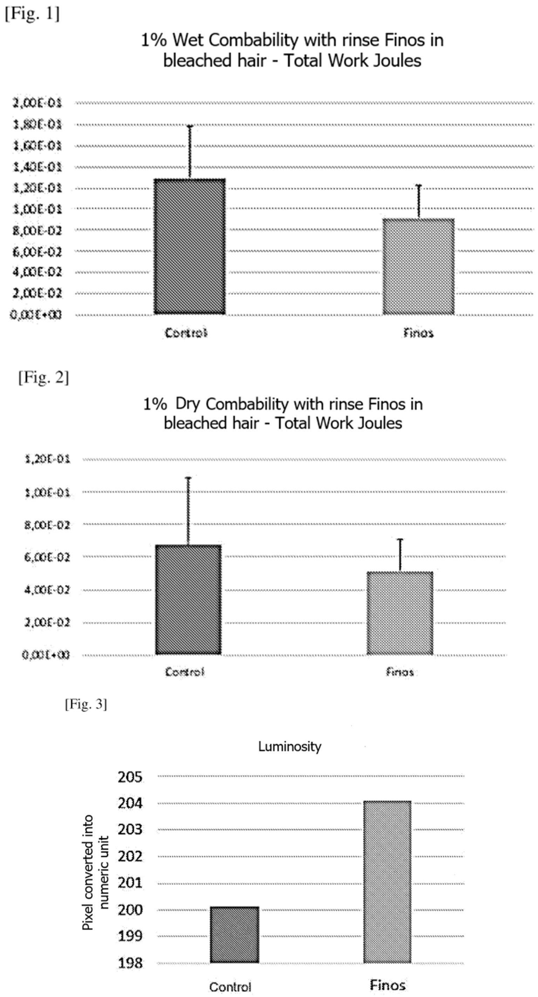

The effect of the cosmetic system of the invention on hair was tested, in comparison to a control sample. A system formulation comprising 1.5% babassu oil and 0.5% buriti oil (Formulation 3 described in example 1) was tested for wet and dry combability. The results can be seen in the graphs of Figure 1 and Figure 2.

Dia-Stron combability analysis has been used as a parameter to qualify and quantify hair fiber combability. The total work for combability of standard bleached hair fibers divided as described for comparative analysis with the Activated Oil product of the invention was measured, with one wet and one dry application.

Data and test conduction:

- Experimental Samples: 6 locks of bleached Caucasian hair of 2.0 grams each were subdivided into 2 groups with triplicate samples as described below:

  - Control: Bleached Caucasian hair, 3 locks were treated, washed only with LESS (Sodium Lauryl Ether Sulfate) at 10%.
  - Activated Oil Finos: Bleached Caucasian hair, 3 locks were treated, washed with LESS at 10%, then treated with 1% Activated Oil Finos 0.5 grams per lock, massaged for 1 minute, and rinsed for 30 seconds.

The procedure was employed to study the changes in total work through the application of the treatments and their comparative effects between the treated and untreated locks.

Experimental conditions:

- Objective: comparatively evaluate the total work of combability and its variations between the groups.
- Damage: Hair Importers Caucasian standard bleached in the laboratory at a standard temperature of 30°C for 40 minutes 2 times, brushed 5 times between bleaching and 10 times between each treatment and air-dried at room temperature.
- Test Conditions: Humidity 53% + 2 and temperature 20°C + 2.

Results: The treated group showed a statistical difference from the control group. It was observed that the product applied 1% Activated Oil Finos promoted a reduction in combability work (total work in Joules), improving hair conditioning both dry and wet.

**Example 4** - **Hair fiber Shine and Luminosity properties**

The effect of treatment with the cosmetic system comprising a combination of oils of the invention, also called Activated Oil Finos version here (babassu oil/buriti oil ratio of 75/25), was tested compared to a control sample on the effect of hair fiber shine.

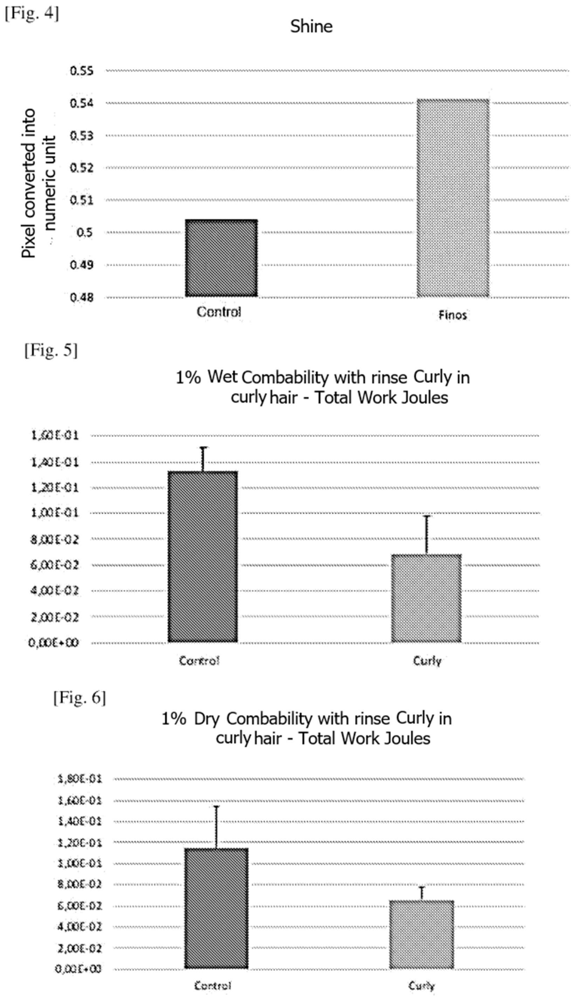

The effect of the cosmetic system of the invention on hair was tested compared to a control sample. A system formulation comprising 1.5% babassu oil and 0.5% buriti oil (Formulation 3 described in example 1) was tested for luminosity and shine. The results can be seen in the graphs of Figure 3 and Figure 4. The unit of measure for the graphs is pixels transformed into integers.

Shine camera analysis has been used as a parameter to qualify and quantify the shine and luminosity of the hair fiber. The total shine and luminosity considered as the intensity at the peak of specular light for the shine of standard straight Caucasian hair fibers divided as described for comparative analysis with the control versus Activated Oil Finos product of the invention were measured.

Data and test conduction:

- Experimental Samples: 2 locks of standard straight Caucasian hair were divided as described below:

  - Control: Straight Caucasian hair washed only with LESS at 10%.
  - Finos: Straight Caucasian hair washed with LESS at 10% then treated with Finos 0.5 grams per lock at 1% massaged for 1 minute and rinsed for 30 seconds.

The procedure was employed to study the changes in shine through the application of the treatment and its comparative effects between the treated and untreated locks.

Experimental conditions:

- Objective: comparatively evaluate the total shine and luminosity between the tested groups.
- Hair: Hair Importers standard straight Caucasian locks.
- Test Conditions: Humidity 53% + 2 and temperature 20°C + 2, shine camera and standardized image processing in image measurement, in 32 bits, following calculation protocol IT.CAR.0024 R00.

- Results: The treated group showed a difference from the control group, positively interfering with both luminosity and shine. It was observed that the sample of the Activated Oil Finos system of the invention showed improvement in the shine and luminosity function compared to the control group.

**Example 5** - **Hair fiber combability force properties**

The effect of treatment with the cosmetic system comprising a combination of oils of the invention, also called Activated Oil Curly version here (coconut oil/baobab oil ratio of 75/25), was tested on the combability force quantified by the total work through Dia-Stron analysis.

The effect of the cosmetic system of the invention on hair was tested compared to a control sample. A system formulation comprising 1.5% coconut oil and 0.5% baobab oil (Formulation 3 described in example 1) was tested for wet and dry combability. The results can be seen in the graphs of Figure 5 and Figure 6.

Dia-Stron combability analysis has been used as a parameter to qualify and quantify hair fiber combability. The total work for combability of standard bleached hair fibers divided as described for comparative analysis with the Activated Oil Curly version product was measured, with one wet and one dry application.

Data and test conduction:

- Experimental Samples: 6 locks of 3C curly Caucasian hair of 2.0 grams each were subdivided into 2 groups with triplicate samples described below:

  - Control: 3C curly Caucasian hair, 3 locks were treated, washed only with LESS at 10%.
  - OA Finos: 3C curly Caucasian hair, 3 locks were treated, washed with LESS at 10% then treated with 1% Activated Oil Curly 0.5 grams per lock, massaged for 1 minute and rinsed for 30 seconds.

The procedure was employed to evaluate the changes in total work through the application of the treatments and their comparative effects between the treated and untreated locks.

Experimental conditions:

- Objective: comparatively evaluate the total work of combability and its variations between the groups.
- Hair: Hair Importers 3C curly Caucasian standard.
- Test Conditions: Humidity 53% + 2 and temperature 20°C + 2.

Results: All groups showed statistical differences from the control group. The treatment groups showed no statistical differences among themselves. It was observed that the product applied 1% Activated Oil Curly promoted a reduction in combability work (total work in Joules), thus improving hair conditioning both dry and wet.

**Example 6** - **Curly hair fiber volume and frizz properties**

The effect of treatment with the cosmetic system comprising a combination of oils of the invention, also called Activated Oil Curly version here (Formulation 3 described in example 1 - coconut oil/baobab oil ratio of 75/25), was tested on the modification of volume and frizz in curly hair.

The analysis of the effect of volume and frizz control, performed through visual assessment of the frizz effect, serves as an illustrative parameter of the changes in this property in the hair fiber. Curly hair and bleached hair tend to present greater hydrophilicity and therefore absorb water more easily and consequently tend to present greater volume and frizz more quickly in ambient humidity. Water absorption by the hair fiber in damaged areas provides greater tangling of the locks and a greater propensity for volume and frizz.

This study was performed as an illustrative parameter of the difference in volume and frizz in visual comparative forms between the sample and the control of non-bleached 3C curly hair versus the same fibers treated with the Activated Oil Curly system of the invention, as a function of time with controlled humidity and temperature.

Experimental Samples: 2 locks of 3C curly hair of 2.0 grams each were subdivided into 2 samples described below:

First, all 3C curly hair locks were washed individually with LESS (10%) 1 ml massaged for 1 minute and rinsed in running water for 1 minute and air-dried naturally for 24 hours175. At this point, the initial measurements were taken. They were then divided, identified, and treated:

- Control: 3C curly hair.
- 1% Curly CP: 3C curly hair treated with 0.5 grams of Styling Cream with 0.5% Activated Oil Curly and massaged for 1 minute.

After 30 minutes, the samples were placed in the oven at 40°C and left overnight.

Experimental conditions:

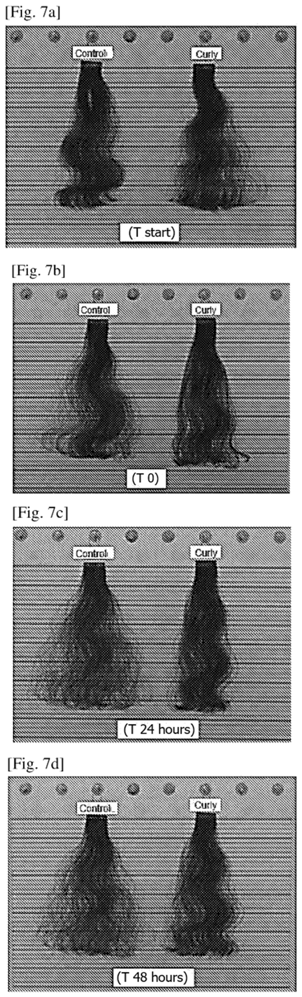

- Objective: compare the efficacy between two treatments on the properties of volume and frizz control in curly hair when exposed to humidity.
- Damage level: non-bleached 3C curly hair.
- Treatment condition: washed with LESS (10%) and treated with 0.50 grams of the described treatments with 1-minute massage and air-dried naturally for 30 minutes at room temperature and overnight in a 40°C oven for 24 hours, then placed in the controlled humidity oven. Protocol IT.CAR.010.R00.
- Test Conditions: The samples were placed at a controlled temperature of 24°C +/- 2°C and 90% humidity, and the measurements were recorded. The results can be observed in Figures 7a-d, where Tstart ([Fig. 7a]) refers to the fibers at the moment before treatment, T0 ([Fig. 7b]) refers to the fibers immediately after treatment, T24 ([Fig. 7c]) refers to the fibers after 24 hours of treatment, and T48 ([Fig. 7d]) refers to the fibers after 48 hours of treatment.

Results: It was observed that the Activated Oil product applied at 1% in water promoted a reduction in frizz in curly hair versus the control at both 24 hours and 48 hours; however, the best performance was at 24 hours.

**Example 7** - **Curly Hair Fiber Volume, Frizz, and Styling Properties**

The effect of a treatment with the cosmetic system comprising a combination of oils of the invention, also called Activated Oil Curly version here (Formulation 3 described in example 1) coconut/baobab - 75/25) was tested on the modification of volume, frizz, and curl styling.

3C curly hair locks were subjected to stress at constant temperature and high humidity level to evaluate the percentage of retained curls over a fixed period of time.

Experimental Samples: 2 locks of 3C curly hair, weighing 2.0 grams each, were subdivided into 2 samples as described below.

First, all locks of curly hair were individually washed with 1 ml of LESS (10%), massaged for 1 minute, rinsed under running water for 1 minute, and naturally dried for 24 hours. Initial measurements were taken at this point. Then, they were divided, identified, and treated as described below:

- Control: 3C Curly Hair
- OA Curly: 3C curly hair treated with 0.5 grams of a 1% water solution of the Activated Oil Curly product of the invention, and massaged for 1 minute.

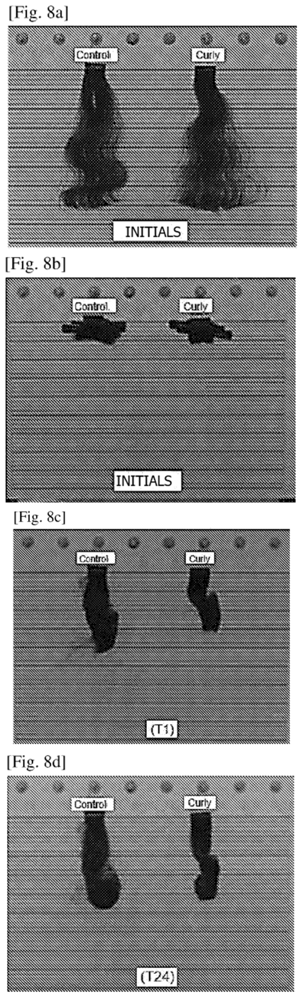

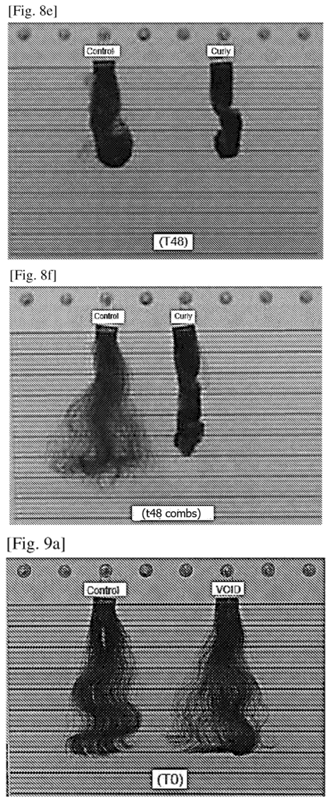

The results can be seen in Figures 8 a-f. Preparation of test samples:

1) The locks were placed on a weighing plate. The measured amount of 0.5 grams of product was added to the locks and the product was massaged through the locks with the fingers.

2) The locks were hung and then combed once to straighten the hair ([Figure 8a]). The hair was rolled diagonally ([Figure 8b]), with the hair coming from the back of the bob to the front of the bob. It was rolled up with an overlap to hold the locks, and continued to be turned upwards in a way that lengthened the locks along the bob until it reached the plane at the top of the lock.

3) The bob was carefully removed and hair clips were placed in place, one in front and one behind. This was repeated for each lock with each product, changing the combs, washing hands and the bob between products.

4) The treated locks were dried overnight in the oven at 40°C. Then the locks were acclimatized for half an hour before being placed on the curl retention holder and placed in the Humidity Chamber for testing ([Figure 8c]).

Experimental conditions:

- Objective: to compare the effectiveness between control and treatment on curl retention, volume, and frizz properties in 3C curly hair when exposed to humidity.
- Damage level: 3C curly hair without bleaching.
- Treatment condition: washed with LESS (10%) and treated with 0.50 grams of the treatments described with a 1-minute massage and natural drying for 30 minutes, at room temperature, and curled according to the described procedure and overnight maintenance in an oven at 40°C for 24 hours, followed by acclimatization for half an hour and placed in a humidity-controlled oven. Protocol IT.CAR.010.R00
- Test conditions: The samples were placed at a controlled temperature of 24°C +/- 2°C and 90% humidity; the recorded measurements were initial (Fig. 8a-c), 24 hours ([Figure 8d]), and 48 hours ([Figure 8e] and [Figure 8f]).
- Calculations of % control and curl retention according to the equation described below:

[Equation 1]

$$\% \text{ Curl Retention} = \frac{(L0) - (Lt)}{(L0) - (Li)} \times 100$$

Where:

LO = Length of stretched hair (cm) = 17.0 cm

Li = Curl length before exposure (cm)
Lt = Curl length after exposure (cm)

[Table 5]

| Sample | L0 (cm) | Li (cm) | L24H (cm) | L48H (cm) | % 24 Horas | % 48 Horas |
|---|---|---|---|---|---|---|
| Control | 17.0 | 1.8 | 13.2 | 14.8 | 25.00 | 14.47 |
| 1% Activated Oil Curly | 17.00 | 1.77 | 11.4 | 11.9 | 36.77 | 33.49 |

Results: The product applied in 1% Activated Oil Curly as the mixture (75/25 ratio of coconut and low oils) was observed to promote frizz reduction in the curly hair versus control of both 24 hours and 48 hours. The locks were combed after the 48 hour test and the wick treated with the 1% Activated Oil Curly showed maintenance of the bunches and appearance of natural curl without frizz compared to the control.

**Example 8 - Volume and Frizz Properties of Curly Hair Fibers**

The treatment effect was tested with the cosmetic system of the invention without the oils, herein also called Void (no oil-free system), in the modification of the volume and frizz in curly hairs.

Analysis of the effect of change of volume and frizz control, made by visual evaluation of the frizz effect, serves as an illustrative parameter of the changes of this property in the hair fiber. Curly hair and discolored hair tend to have greater hydrophilicity and thereby absorb water more easily and therefore tend to have greater volume and frizz with greater speed and ambient moisture. The absorption of water by the hair fiber over the damaged areas provides greater bowing of the locks and greater propensity for bulk and frizz.

This study was performed as an illustrative parameter of the volume difference and frizz of comparative forms visually between the samples and control of curly hairs without discoloration versus the same fibers treated with the samples as a function of time with controlled humidity and temperature.

Experimental Samples: 2 hair locks of 2.0 grams each were subdivided into 2 samples as described below.

First all of the hair locks 3C were individually washed with LESS (10%) 1 ml, massaged for 1 minute and rinsed in running water for 1 minute, and allowed to dry at t for 24 hours. At this point initial measurements were made.

The following were split, identified and treated described below:

- Control: Curly hair 3C
- 1% Void: Curly hair 3C treated with 0.5 grams of Activated Oil Void aqueous solution and massaged for 1 minute. Void is the name of the system identical to Curly but without the vegetable oils, i.e. the empty micelle.

After 30 minutes the samples were placed in the oven at 40°C and left overnight.

Experimental conditions:

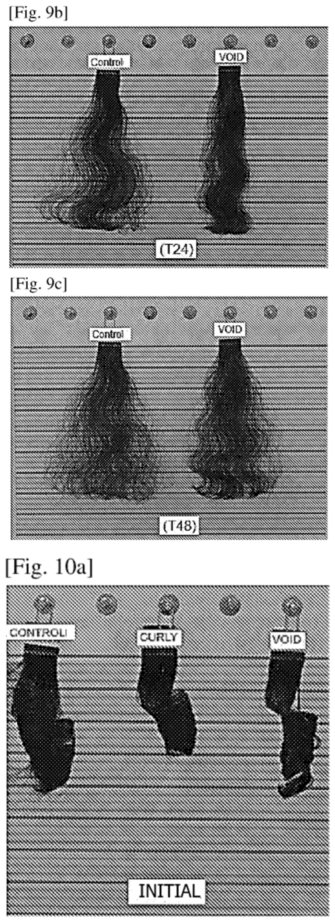

- Objective: compare the efficacy between treatment and control in the Volume and Frizz properties on the curly hair when exposed to moisture.
- Level of Damage: Curly hair 3C without discoloring.
- Treatment condition: washed with LESS (10%) and treated with 0.50 grams of the treatment described with massage of 1 min and dried naturally 30 minutes at room temperature and overnight at 40°C in a stove for 24 hours then placed in the controlled moisture stove. Protocol IT.CAR.010.R00.
- Test conditions: The samples were placed at the controlled temperature of 24°C +/- 2°C and 90% humidity as the recorded measurements were initial ([Figure 9a]), 24 hours (Figure 9b) and 48 hours ([Figure 9c]). The results can be seen in Figures. 9a-c.

Results: The sample was observed to show reduced frizz and volume both within 24 hours and 48 hours compared to the control.

**Example 9** - **Volume, Frizz and Modelling Properties of the Curly Hair Fibers.**

The effect of a treatment with the cosmetic system was tested without the combination of oils of the invention, herein also called Void (no oil-free system), in volume modification, frizz and bunch modeling.

Hair locks 3C were subjected to stress at constant temperature and high moisture level to assess the percentage of bunches retained for a fixed period of time. Modeling products are sensitive to the initial drop-in-drop of curvature, therefore length measurements were annotated at 30 minutes, 24 hours, and 48 hours. Calculations were performed to evaluate the initial bunch drop over 30 minutes as well as the total amount of bunches retained after 24 and 48 hours in the high humidity environment. For this test, the bunches were used together with the frizz in an attempt to understand the effect of the product on the maintenance and shaping of bunches in the presence of moisture and frizz in the presence of moisture near the bunch.

Experimental Samples: 2 Hair locks 3C of 2.0 grams each were subdivided into 2 samples described below.

First all of the hair locks were individually washed with LESS (10%) 1 ml, massaged for 1 minute and rinsed in running water for 1 minute and allowed to dry at t for 24 hours. At this point initial measurements were made. The following was divided and treated described below in:

- Control: Curly hair 3C
- Void: Curly hair 3C treated with 0.5 grams of 1% solution in water of the Void product and massaged for 1 minute.

Preparation of Test Samples:

1) The locks were placed in the weigh pan. The measured amount of 0.5 grams of product was added to the locks and the product was massaged through the locks with the fingers.

2) The locks were hung up and then combed once to straighten the hair.. The hair was wound diagonally, with hair from the back of bob to bob in front of bob. The locks were wound up with an overlap to hold the locks, it continued to turn upwards in a manner that lengthens the locks along the bob until they reach the plane at the top of the lock.

3) Bob was carefully removed and hair clips were put in place, one at the front and the other behind. The process was repeated for each lock with each product, changing the combs, washing hands and the roller between products..

4) The treated locks were dried overnight in an oven at 40°C. Then, the locks were allowed to acclimate for half an hour before being placed on the curl retention support and then in the humidity chamber for testing.

Experimental conditions:

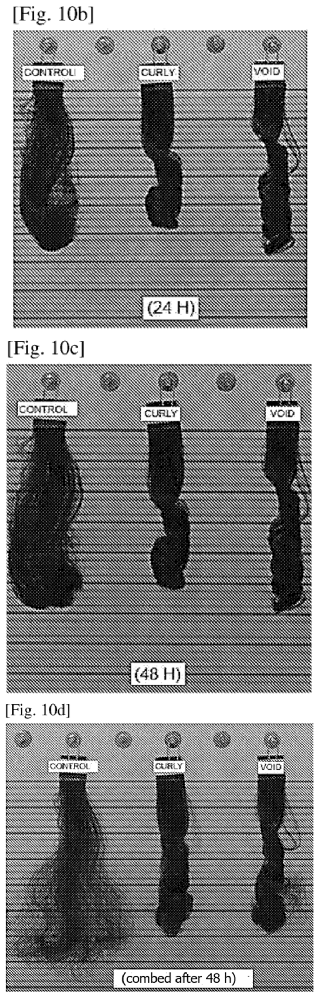

- Objective: to compare the effectiveness between control and treatment on the properties of curl retention, volume, and frizz in curly hair 3C when exposed to humidity.
- Damage level: curly hair 3C without bleaching.
- Treatment condition: washed with LESS (10%) and treated with 0.50 grams of the treatments described with massage of 1 minute and natural drying for 30 minutes at room temperature and wound according to the procedure described and maintenance overnight in an oven at 40°C for 24 hours, followed by half an hour environment and placed in the controlled moisture stoven. Protocol IT.CAR.010.R00
- Test conditions: Samples were placed at the controlled temperature of 24°C +/- 2°C and humidity 90%, the recorded measurements were initial, 24 hours and 48 hours. The results can be seen in Figures 10 to-d.
- Calculations of % control and bunch retention according to the equation described below::

[Equation 2]

$$\% \text{ Curl Retention} = \frac{(L0) - (Lt)}{(L0) - (Li)} \times 100$$

Where:

$$L0 = \text{Stretched hair length (cm)} = 17.0 \text{ cm}$$

Li = Bunch Length before exposure (cm)
Lt = Bunch Length after exposure (cm)

[Table 6]

| Sample | L0 (cm) | Li (cm) | L24H (cm) | L48H (cm) | % 24 Horas | % 48 Horas |
|---|---|---|---|---|---|---|
| Control | 17.0 | 1.8 | 13.2 | 14.8 | 25.00 | 14.47 |
| 1% Activated oil Curly | 17.00 | 1.77 | 13.5 | 14.7 | 22.98 | 15.11 |

Results: The void product was observed to promote reduction after 48 hours. The locks were combed after the 48-hour test and the locks treated with the 1% Void treatment showed maintenance of the locks and appearance of natural curl without frizz compared to the control.

**Example 10 - Shine and Luminosity Properties of the Hair Fiber**

The effect of a treatment with the cosmetic system without the combination of oils of the invention, herein also called Void, has been tested, in the effect of the shine and luminosity of the hair fiber. The effect of the cosmetic system of the invention on hair was tested as compared to a control sample.

The analysis of the luminosity camera has been used as a parameter to qualify and quantify the shine and luminosity of the hair fiber. Total shine and luminosity were measured, considered as intensity at the peak of specular light for the shine of standard Caucasian straight hair fibers split as described for comparative analysis with the control *versus* Activated Oil Void (without oils), with an application of 1% system diluted in water.

Experimental Samples: 2 standard Caucasian straight hair locks were subdivided into groups as described below:

- Control: Caucasian straight hair washed with only 10% LESS.
- Void: Caucasian straight hair washed with 10% LESS then treated with Void (no oils) 0.5 grams per wick to 1% massaged for 1 minute and rinsed for 30 seconds.

The procedure was used to study the shine changes by applying the treatments and their comparative effects between the treated and untreated locks.

Experimental conditions:

- Objective: To compare the total shine and luminosity among the groups.
- Hair: standard Caucasian straight hair locks Hair Imports
- Test Conditions: Moisture 53% + 2 and temperature 20°C +2, shinechamber and standard image treatment as measured in the image, in 32 bits, following the calculation protocol IT.CAR..000024 R00.

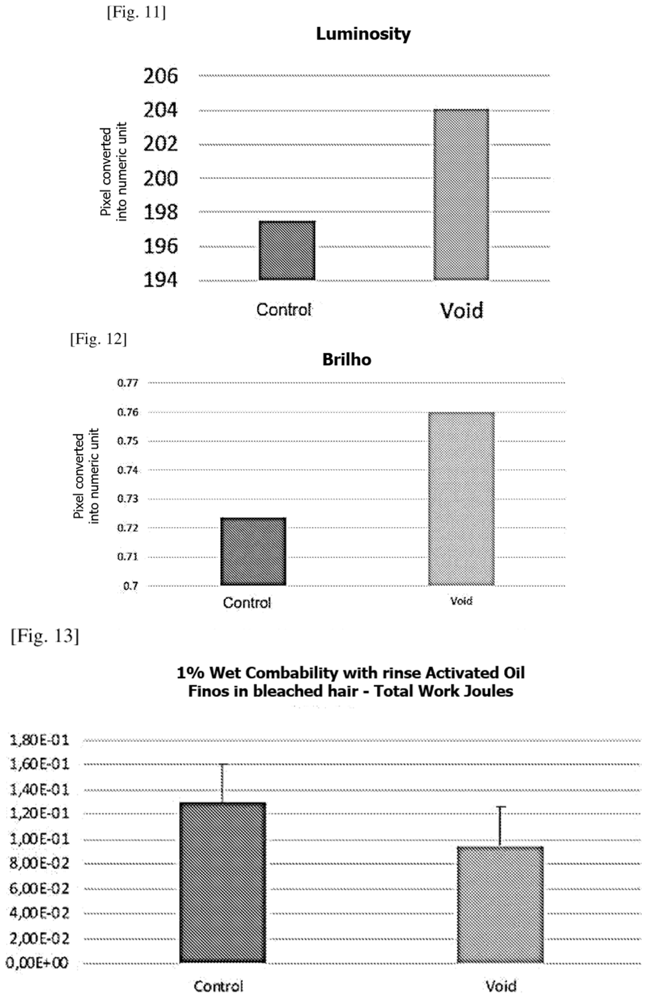

Results: The treated group showed a difference with the control group, positively interfering with both shine and luminosity. It was observed that the sample treated with the system showed improvement of the shine and luminosity function compared to the Control. The results can be seen in the graphs of Figure 11 and Figure 12. The measure unit of the graphs is transformed pixels into numbers.

**Example 11 - Combability strength properties of hair fibers**

The effect of a treatment with the active cosmetic system was tested without the combination of oils of the invention, herein also called Void (non-oil structure), in the total workability force by Day-Stron analysis. The effect of the cosmetic system of the invention was tested as compared to a control sample.

Penteability analysis via Dia-Stron has been used as a parameter to qualify and quantify the combability of the hair fiber. Total work for the combability of the divided bleached standard hair fibers was measured as described for comparative analysis with the product Activated Oil Void (without oils), with a wet and dry application.

Experimental Samples: Six locks of bleached Caucasian hair, weighing 2.0 grams each, were subdivided into two groups with triplicate samples described below:

- Control: 3 bleached Caucasian hair locks were treated, washed only with 10% LESS.
- OA Void: 3 bleached Caucasian hair locks were treated with 10% LESS to followed by treating with Activated Oil Void (no oils), 0.5 grams per lock at 1%, massaged for 1 minute and rinsed for 30 seconds.

The procedure was used to study changes in total work by applying treatments and their comparative effects between treated and untreated locks.

Experimental conditions:

- Objective: to evaluate in comparison the overall workability and their variations among groups.
- Damage: standard caucasian Hair Importers bleached in laboratory at standard temperature of 30°C for 40 minutes 2 times, brushed 5 times between discolorations and 10 times between each treatment and dried at room temperature
- Test Conditions: Moisture 53% + 2 and temperature 20°C + 2.

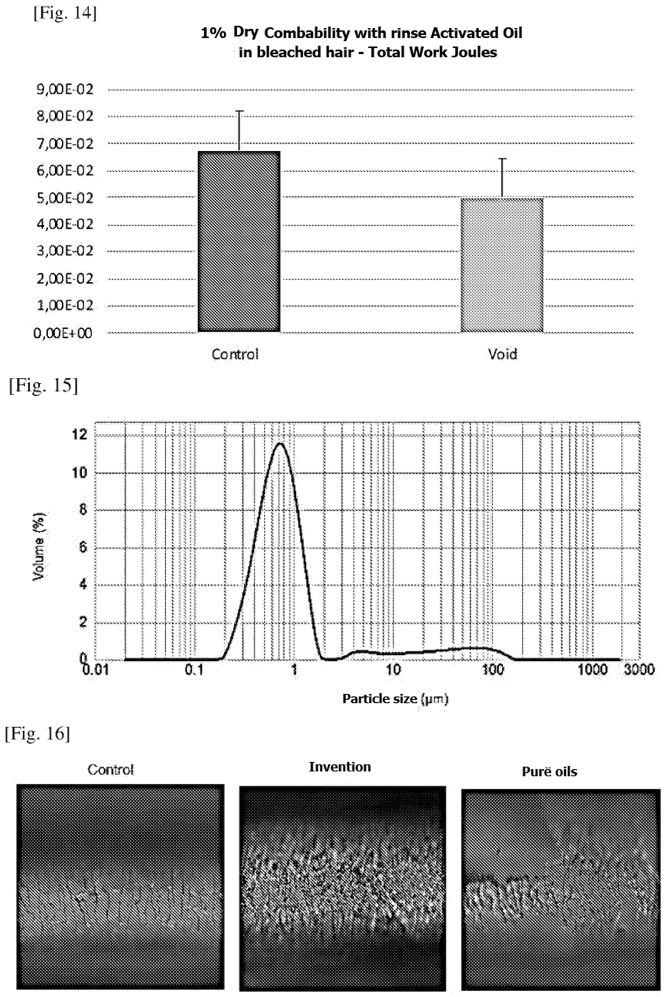

Results: The treated group showed statistical differences with the control group. It was observed that the product applied 1% Activated Oil Void promoted reduced workability work (total work in Joules), improving dry and wet hair conditioning. The results can be seen in the graphs of Figure. 13 and Figure 14.

**Example 12 - Particle Properties**

An active cosmetic system of the invention (formulation 3 comprising a combination of buriti oil and babassu) was analyzed for particle size and distribution determination.

The graph of Figure 15 represents the measurement of particle size and distribution made by dilution method 1:10 with laser measurements with Malvern equipment.

Analysis of comparative microscopies between different systems (control, active system of the invention comprising combination of the vegetable oils buriti and babassu and pure vegetable oils) is represented in Figure 16.

The image methodology of the systems of the systems (Figure 16) comprises 50 times magnification with optical microscopy using polarized light. It is known that oils are not microemulsions, as soon as viewed in microscopy directly would not be seen in polarized light.

**Claims**

1. ACTIVE COSMETIC SYSTEM, **characterized by** comprising at least one emulsifying agent, at least one conditioning agent and at least one humectant agent.

2. ACTIVE COSMETIC SYSTEM, according to claim 1, **characterized in that** it comprises a primary emulsifying agent and a secondary emulsifying agent.

3. ACTIVE COSMETIC SYSTEM, according to claim 1 or 2, **characterized in that** it comprises:

   - about 0.5-11%, preferably 1-6%, based on the total weight of the system, of at least one emulsifying agent;
   - about 1-5%, preferably 2-4%, based on the total weight of the system, of at least one conditioning agent; and
   - about 80-97%, preferably 90-95%, based on the total weight of the system, of at least one humectant agent.

4. ACTIVE COSMETIC SYSTEM, according to claim 3, **characterized in that** it comprises:

   - 2-4%, based on the total weight of the system, of a primary emulsifying agent;
   - 1-2%, based on the total weight of the system, of a secondary emulsifying agent;
   - 2-4%, based on the total weight of the system, of a conditioning agent; and
   - 90-95%, based on the total weight of the system, of a humectant agent.

5. ACTIVE COSMETIC SYSTEM, according to any one of claims 1 to 4, **characterized in that** it is in the form of a microemulsion.

6. ACTIVE COSMETIC SYSTEM, according to any one of claims 1 to 5, **characterized in that** the primary emulsifying agent is selected from the group comprising sucrose stearate, lauryl sucrose, cetyl sucrose, 100% plant-derived compounds such as sorbitan olivate, polyglyceryl-6 olivate, polyglyceryl compounds, lecithin, or a mixture thereof, the secondary emulsifying agent is selected from the group comprising hydrolyzed wheat protein, hydrolyzed rice protein, soy extract or derivative, hydrolyzed soy protein, glyceryl/octyldodecanol soy oil ester, or a mixture thereof, the conditioning agent is selected from the group comprising fatty acid ester derivatives, such as vegetable oils, vegetable butters, proteins, and quaternary compounds such as quaternium 22, or a mixture thereof.

7. ACTIVE COSMETIC SYSTEM, according to any one of claims 1 to 6, **characterized by** additionally comprising about 0.5-3.0% by weight based on the total weight of the system, of saturated and unsaturated vegetable oils.

8. ACTIVE COSMETIC SYSTEM, according to claim 7, **characterized in that** the ratio of unsaturated oils to saturated oils in the composition of the cosmetic system of the present invention varies from 10:90, particularly from 40:60, preferably 25:75.

9. ACTIVE COSMETIC SYSTEM, according to claim 7 or 8, **characterized in that** the saturated vegetable oils are selected from coconut oils and babassu oils, or a combination thereof, and the unsaturated vegetable oils are selected from baobab oil, buriti oil, and sesame oil, or a combination of two or more thereof.

10. USE OF THE ACTIVE COSMETIC SYSTEM, as defined in any one of claims 1 to 9, **characterized in that** it is in the preparation of cosmetic compositions for hair and/or scalp care.

11. COSMETIC COMPOSITION, **characterized by** comprising an active cosmetic system, as defined in any one of claims 1 to 9, and cosmetically acceptable ingredients.

12. COSMETIC COMPOSITION, according to claim 11, **characterized in that** it is a cream, a lotion, soap, shampoo, conditioner, hair mask, leave-on product, and/or split end repairer.

13. METHOD FOR THE COSMETIC TREATMENT OF HAIR FIBERS AND/OR THE SCALP, **characterized by** comprising the application of the active system, as defined in any one of claims 1 to 9, or a cosmetic composition, as defined in claim 11 or 12, onto the hair fibers and/or the scalp.

[Fig. 1]

1% Wet Combability with rinse Finos in bleached hair - Total Work Joules

[Fig. 2]

1% Dry Combability with rinse Finos in bleached hair - Total Work Joules

[Fig. 3]

Luminosity

[Fig. 4]

Shine

[Fig. 5]

1% Wet Combability with rinse Curly in curly hair - Total Work Joules

[Fig. 6]

1% Dry Combability with rinse Curly in curly hair - Total Work Joules

[Fig. 7a]

(T start)

[Fig. 7b]

(T 0)

[Fig. 7c]

(T 24 hours)

[Fig. 7d]

(T 48 hours)

[Fig. 8a]

[Fig. 8b]

[Fig. 8c]

[Fig. 8d]

[Fig. 8e]

[Fig. 8f]

[Fig. 9a]

[Fig. 9b]

(T24)

[Fig. 9c]

(T48)

[Fig. 10a]

INITIAL

[Fig. 10b]

(24 H)

[Fig. 10c]

(48 H)

[Fig. 10d]

(combed after 48 h)

[Fig. 11]

## Luminosity

[Fig. 12]

## Brilho

[Fig. 13]

## 1% Wet Combability with rinse Activated Oil Finos in bleached hair - Total Work Joules

[Fig. 14]

**1% Dry Combability with rinse Activated Oil in bleached hair - Total Work Joules**

[Fig. 15]

[Fig. 16]

Control · Invention · Purë oils

# EP 4 732 828 A1

## INTERNATIONAL SEARCH REPORT

International application No.

**PCT/BR2024/050212**

### A. CLASSIFICATION OF SUBJECT MATTER

**A61K 8/92**(2006.01)i; *A61K 8/34*(2006.01)i; *A61K 8/84*(2006.01)i; *A61K 8/41*(2006.01); *A61K 8/60*(2006.01); A61Q 5/12(2006.01)
CPC: A61K 8/068; A61K 8/345; A61K 8/922; A61K 8/41; A61K 8/60; A61Q 5/12; A61K 2800/412

According to International Patent Classification (IPC) or to both national classification and IPC

### B. FIELDS SEARCHED

Minimum documentation searched (classification system followed by classification symbols)

A61K 8/92; A61K 8/34; A61K 8/84; A61K 8/41; A61K 8/60; A61Q 5/12

Documentation searched other than minimum documentation to the extent that such documents are included in the fields searched

INPI - BR BANCO DE PATENTES

Electronic data base consulted during the international search (name of data base and, where practicable, search terms used)

DERWENT

### C. DOCUMENTS CONSIDERED TO BE RELEVANT

| Category* | Citation of document, with indication, where appropriate, of the relevant passages | Relevant to claim No. |
|---|---|---|
| X | WO 2022094731 A1 (EVERIST INC [CA]) 12 May 2022 (2022-05-12) | 1-2 |
| A | The whole document | 3-13 |
| X | EP 3478254 B1 (DOW BRASIL SUDESTE IND LTDA [BR]) 28 December 2022 (2022-12-28) | 1 |
| A | The whole document | 2-13 |
| X | BR 112022016767 A2 (CRODA INC [US]) 11 October 2022 (2022-10-11) | 1 |
| A | The whole document | 2-13 |

☐ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 June 2024** | **15 July 2024** |

| Name and mailing address of the ISA/BR | Authorized officer |
|---|---|
| **National Institute of Industrial Property (Brazil)** **Rua Mayrink Veiga 9, 20° andar, Centro Rio de Janeiro RJ – CEP 20.090-910** **Brazil** | **RENATA ENGELHARDT** |
| Telephone No. **(55 21) 3037-3742, 3037-3984** | Telephone No. **+55 21 3037 4528 - 3037 3319** |

Form PCT/ISA/210 (second sheet) (July 2022)

24

**INTERNATIONAL SEARCH REPORT**
Information on patent family members

International application No.

**PCT/BR2024/050212**

| Patent document cited in search report | | | Publication date (day/month/year) | Patent family member(s) | | | Publication date (day/month/year) |
|---|---|---|---|---|---|---|---|
| WO | 2022094731 | A1 | 12 May 2022 | CA | 3197289 | A1 | 12 May 2022 |
| | | | | EP | 4240310 | A1 | 13 September 2023 |
| | | | | US | 2023338257 | A1 | 26 October 2023 |
| EP | 3478254 | B1 | 28 December 2022 | EP | 3478254 | A1 | 08 May 2019 |
| | | | | AR | 109458 | A1 | 12 December 2018 |
| | | | | BR | 112018076752 | A2 | 26 March 2019 |
| | | | | BR | 112018076752 | B1 | 03 March 2022 |
| | | | | CN | 110167518 | A | 23 August 2019 |
| | | | | CN | 110167518 | B | 03 May 2022 |
| | | | | JP | 2019519555 | A | 11 July 2019 |
| | | | | JP | 7017527 | B2 | 08 February 2022 |
| | | | | US | 2019192402 | A1 | 27 June 2019 |
| | | | | US | 11071702 | B2 | 27 July 2021 |
| | | | | WO | 2018000068 | A1 | 04 January 2018 |
| BR | 112022016767 | A2 | 11 October 2022 | CA | 3168373 | A1 | 02 September 2021 |
| | | | | CN | 115175661 | A | 11 October 2022 |
| | | | | EP | 4110282 | A1 | 04 January 2023 |
| | | | | JP | 2023516168 | A | 18 April 2023 |
| | | | | US | 2023094014 | A1 | 30 March 2023 |
| | | | | WO | 2021173954 | A1 | 02 September 2021 |
| | | | | WO | 2021173954 | A9 | 09 December 2021 |
| | | | | ZA | 202208996 | B | 31 May 2023 |

Form PCT/ISA/210 (patent family annex) (July 2022)